# EUROPÄISCHE PATENTANMELDUNG

(11) **EP 3 366 671 A1**
(43) Veröffentlichungstag der Anmeldung: **29.08.2018**
(21) Anmeldenummer: 17158522.7
(22) Anmeldetag: 28.02.2017
(51) Int. Cl.: C07C 303/04, C07C 309/22

(54) **VERFAHREN ZUR HERSTELLUNG VON SULFOBERNSTEINSÄURE UND VERWENDUNG VON SOLFOBERNSTEINSÄURE**

(71) Anmelder: ESIM Chemicals GmbH, 4020 Linz (AT)
(72) Erfinder: STÖGLEHNER, Alex, 4210 Gallneukirchen (AT); KASSLER, Alexander, 1150 Wien (AT); KÖNIG, Michael, 4020 Linz (AT); KOGLER, Martina, 4030 Linz (AT); HINTERWIRTH, Helmut, 4020 Linz (AT); SCHUECKER, Raffael, 4020 Linz (AT); HÄUBL, Martin, 4040 Linz (AT); DOMKE, Lutz, 4020 Linz (AT); HOLUB, Bernhard, 4072 Strassham (AT)
(74) Vertreter: Prüfer & Partner mbB Patentanwälte · Rechtsanwälte

(57) **Zusammenfassung**

Verfahren zum Herstellen von Sulfobernsteinsäure und davon abgeleiteten Verbindungen durch Umsetzen von Fumarsäure, Maleinsäure, Maleinsäureanhydrid und von diesen Verbindungen abgeleiteten Verbindungen mit Schwefeldioxid in Gegenwart von Wasser.

## Beschreibung

Vorliegende Erfindung bezieht sich auf ein Verfahren zum Herstellen von Sulfobernsteinsäure und davon abgeleiteten Verbindungen sowie die Verwendungen damit hergestellter Sulfobernsteinsäure oder davon abgeleiteter Verbindungen.

Verfahren zum Herstellen von Sulfobernsteinsäure und davon abgeleiteten Verbindungen durch Umsetzung von Maleinsäure, Maleinsäureanhydrid oder Fumarsäure bzw. deren Derivaten (beispielsweise Ester der genannten Säuren) in wässrigem Milieu mit Sulfitsalzen (Sulfite, Hydorgensulfite, Disulfite von Metallen) sind im Stand der Technik bekannt.

Die DE 25 07 520 A1 offenbart ein Verfahren, in welchem ein Maleinsäuremonoalkylester mit Natriumsulfit zur Reaktion gebracht und der entsprechende Natriumsulfosuccinatmonoester erhalten wird.

Die US 5,543,555 A offenbart ein Verfahren, in welchem ein Maleinsäurediester mit einer Mischung aus Natriumdisulfit und Natriumsulfit in wässrigem Milieu zur Reaktion gebracht und der entsprechende Natriumsulfosuccinatdiester erhalten wird.

Die JP 2010064987 A offenbart ein Verfahren in welchem Fumarsäure mit unterschiedlichen Mischungen von Natriumdisulfit und Natriumsulfit zur Reaktion gebracht und das entsprechende Natriumsulfosuccinat erhalten wird.

Die Reaktionsprodukte dieser Verfahren fallen als Salze mit Metallkationen, insbesondere als Alkalimetall- oder Erdalkalimetallsalze an. Um in weiterer Folge die freie Säureform der Sulfobernsteinsäure bzw. der entsprechenden Derivate, beispielsweise Ester, zu erhalten, wird durch zusätzliche Reinigungsschritte wie durch Einsatz eines Ionenaustauschers das Alkalimetallion (bzw. bei Einsatz von Sulfitsalzen anderer Metalle die entsprechenden Kationen) gegen Wasserstoff ausgetauscht.

Als separater Reaktionsschritt wird in der JP 2010064987 A die Herstellung der freien Säureform der Sulfobernsteinsäure ausgehend von Natriumsulfosuccinat durch die Verwendung von sauren Ionenaustauschern beschrieben.

Der im Stand der Technik bekannte Einsatz von Ionenaustauschern ist zeitintensiv und erfordert große Mengen an Lösemitteln zur Regeneration und Konditionierung. Die Entsorgung der dabei entstehenden Abfälle (beispielsweise Abwässer) ist außerdem ressourcen- und kostenintensiv.

Darüber hinaus ist die Entfernung von Ionen mittels Ionenaustauschern oft unvollständig, so dass die mittels der im Stand der Technik erhaltene Sulfobernsteinsäure oft einen Ionengehalt aufweist, der für viele Verwendungen der Sulfobernsteinsäure, beispielsweise für die Verwendung als Quervernetzter zur Herstellung eines Absorbermaterials, zu hoch ist, da ionische Verunreinigungen bei vielen Verwendungen störend sind.

Eine Aufgabe der vorliegenden Erfindung besteht in der Bereitstellung eines verbesserten Verfahrens zur Herstellung von Sulfobernsteinsäure und davon abgeleiteten Verbindungen.

Die Aufgabe wird gelöst durch ein Verfahren gemäß Anspruch 1.

Insbesondere sind mittels des erfindungsgemäßen Verfahrens hergestellte Sulfobernsteinsäure oder davon abgeleitete Verbindungen sind frei oder im Wesentlichen frei von Alkalimetallionen und gegebenenfalls frei oder im Wesentlichen frei von Erdalkalimetallionen oder anderen Verunreinigungen durch Kationen. Daher eignen sich mittels dieses Verfahrens hergestellte Sulfobernsteinsäure oder davon abgeleitete Verbindungen besonders für Anwendungen, in denen es auf eine definierte Konzentration an Alkalimetallionen und gegebenenfalls anderen Kationen ankommt, insbesondere für Verwendungen gemäß Anspruch 12. Darüber hinaus sind durch das Verfahren gemäß Anspruch 13 spezielle, von Derivaten der Sulfobernsteinsäure abgeleitete Salze zugänglich. Diese Salze eignen sich insbesondere für eine Verwendung gemäß Anspruch 14.

Die in den jeweiligen Unteransprüchen festgelegten Merkmale und die in der Beschreibung angeführten Merkmale stellen Weiterbildungen des in den unabhängigen Ansprüchen definierten Lösungsprinzips dar und tragen jeweils weiter zur Erreichung der überraschenden Effekte und weiterer unerwarteter Vorteile bei, die nachfolgend beschrieben werden. Erfindungsgemäß wird eine Verbindung der Formel **I** durch Umsetzen mindestens einer aus der aus den Verbindungen mit den Formeln **II**, **III** und **IV** bestehenden Gruppe ausgewählten Verbindung mit Schwefeldioxid in Gegenwart von Wasser erhalten.

Die Formeln **I, II**, **III**, **IV** sind nachfolgend angegeben. Diese Definitionen gelten für die gesamte Beschreibung.

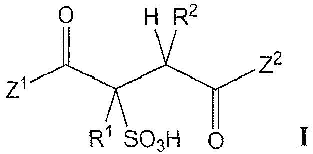

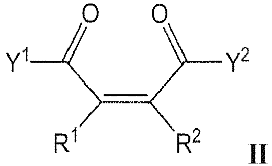

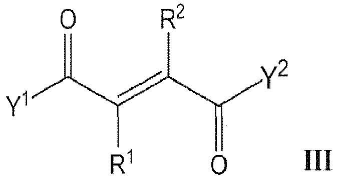

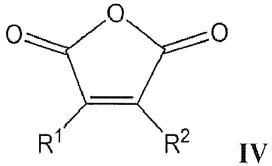

Die Substituenten -R¹, -R², -Z¹, -Z², Y¹ und -Y² sind nachfolgend definiert. Diese Definitionen gelten für die gesamte Beschreibung.

Der Substituent -R¹ wird aus der aus -H und Alkyl bestehenden Gruppe ausgewählt. Der Substituent -R² wird ebenfalls aus der aus -H und Alkyl bestehenden Gruppe ausgewählt. Die Auswahl des Substituenten -R² erfolgt dabei unabhängig von der Auswahl des Substituenten -R¹.

Der Substituent -Z¹ wird aus der aus -OH, -OR¹¹, -NH₂, -NHR¹², -NHR¹²R¹³ und -Cl bestehenden Gruppe ausgewählt. Der Substituent -Z² wird aus der aus -OH, -OR²¹, -NH₂,-NHR²², -NHR²²R²³ und -Cl bestehenden Gruppe ausgewählt. Die Auswahl des Substituenten -Z² erfolgt dabei unabhängig von der Auswahl des Substituenten -Z¹.

Der Substituent -Y¹ wird aus der aus -OH, -OR¹¹, -NH₂, -NHR¹², -NHR¹²R¹³ und -Cl bestehenden Gruppe ausgewählt. Der Substituent -Y² wird aus der aus -OH, -OR²¹, -NH₂,-NHR²², -NHR²²R²³ und -Cl bestehenden Gruppe ausgewählt. Die Auswahl des Substituenten -Y² erfolgt dabei unabhängig von der Auswahl des Substituenten -Y¹.

Die Substituenten -R¹¹, -R¹², -R¹³, -R²¹, -R²², -R²³ stehen jeweils für einen Alkylrest.

Gemäß einer bevorzugten Ausführungsform der Erfindung handelt es sich bei -R¹, -R², -R¹¹,-R¹², -R¹³, -R²¹, -R²², -R²³ um kurzkettige Alkylreste, insbesondere um Alkylreste mit bis zu sechs Kohlenstoffatomen.

Gemäß einer weiteren bevorzugten Ausführungsform der Erfindung wird der Substituent -R¹ aus der aus -H, Methyl, Ethyl, *n*-Propyl, *iso*-Propyl, *n*-Butyl, *iso*-Butyl, *sec*-Butyl und *tert-*Butyl bestehenden Gruppe ausgewählt.

Gemäß einer weiteren bevorzugten Ausführungsform der Erfindung wird der Substituent -R² aus der aus -H, Methyl, Ethyl, *n*-Propyl, *iso*-Propyl, *n*-Butyl, *iso*-Butyl, *sec*-Butyl und *tert-*Butyl bestehenden Gruppe ausgewählt.

Gemäß einer weiteren bevorzugten Ausführungsform der Erfindung werden die Substituenten -R¹ und -R² so gewählt, dass -R¹ = -R² = -H.

Gemäß einer weiteren bevorzugten Ausführungsform der Erfindung werden -R¹¹, -R¹², -R¹³,-R²¹, -R²², -R²³ jeweils und unabhängig voneinander aus der aus Methyl, Ethyl, *n*-Propyl, *iso-*Propyl, *n*-butyl, *iso*-Butyl, *sec*-Butyl und *tert*-Butyl bestehenden Gruppe ausgewählt.

Gemäß einer weiteren bevorzugten Ausführungsform der Erfindung wird der Substituent -Y¹ aus der aus -OH und -OR¹¹ bestehenden Gruppe ausgewählt.

Gemäß einer weiteren bevorzugten Ausführungsform der Erfindung ist der Substituent -Y¹ eine Hydroxygruppe.

Gemäß einer weiteren bevorzugten Ausführungsform der Erfindung wird der Substituent -Y² aus der aus -OH und -OR²¹ bestehenden Gruppe ausgewählt.

Gemäß einer weiteren bevorzugten Ausführungsform der Erfindung ist der Substituent -Y² eine Hydroxygruppe.

Gemäß einer weiteren bevorzugten Ausführungsform der Erfindung liegt die Verbindung mit der Formel **I** nach ihrer Herstellung in Form einer wässrigen Lösung vor.

Gemäß einer weiteren bevorzugten Ausführungsform der Erfindung beträgt die Temperatur bei der Umsetzung mindestens 0 °C, bevorzugt mindestens 10 °C, noch mehr bevorzugt mindestens 20 °C.

Gemäß einer weiteren bevorzugten Ausführungsform der Erfindung beträgt die Temperatur bei der Umsetzung höchstens 250 °C, mehr bevorzugt höchstens 220 °C, noch mehr bevorzugt höchstens 190 °C.

Gemäß einer weiteren bevorzugten Ausführungsform der Erfindung umfasst das erfindungsgemäße Verfahren folgende Schritte:
- Bereitstellen von Wasser;
- optional Lösen von Schwefeldioxid im Wasser;
- Beimengen mindestens einer aus der aus den Verbindungen mit den Formeln **II, III** und **IV** bestehenden Gruppe ausgewählten Verbindung;
- wobei -R¹, -R², -Y¹ und -Y² wie obenstehend definiert sind;
- Einleiten von Schwefeldioxid.

Gemäß einer weiteren bevorzugten Ausführungsform der Erfindung wird das Schwefeldioxid in Form von Schwefeldioxidgas und/oder flüssigem Schwefeldioxid eingeleitet, wobei das Einleiten bevorzugt unter Überdruck erfolgt.

Gemäß einer weiteren bevorzugten Ausführungsform der Erfindung beträgt für die Umsetzung das Verhältnis der Stoffmenge des eingesetzten Schwefeldioxids und der Stoffmenge der eingesetzten aus den Verbindungen mit den Formeln **II, III** und **IV** bestehenden Gruppe ausgewählten Verbindung höchstens 1000, bevorzugt höchstens 500, mehr bevorzugt höchstens 100.

Gemäß einer weiteren bevorzugten Ausführungsform der Erfindung beträgt für die Umsetzung das Verhältnis der Stoffmenge des eingesetzten Schwefeldioxids und der Stoffmenge der eingesetzten aus den Verbindungen mit den Formeln **II**, **III** und **IV** bestehenden Gruppe ausgewählten Verbindung mindestens 1, bevorzugt mindestens 5, mehr bevorzugt mindestens 10.

Gemäß einer weiteren bevorzugten Ausführungsform der Erfindung wird die mindestens eine aus der aus den Verbindungen mit den Formeln **II**, **III** und **IV** bestehenden Gruppe ausgewählte Verbindung zumindest teilweise in Form einer wässrigen Lösung beigemengt.

Gemäß einer weiteren bevorzugten Ausführungsform der Erfindung wird die mindestens eine aus der aus den Verbindungen mit den Formeln **II**, **III** und **IV** bestehenden Gruppe ausgewählte Verbindung zumindest teilweise in Form eines Feststoffs und/oder in Form einer Schmelze beigemengt.

Gemäß einer weiteren bevorzugten Ausführungsform der Erfindung umfasst das erfindungsgemäße Verfahren folgende Schritte:
- Bereitstellen mindestens einer aus den Verbindungen mit den Formeln **II**, **III** und **IV** bestehenden Gruppe ausgewählten Verbindung, die zumindest teilweise als Schmelze vorliegt, bevorzugt Bereitstellen von Maleinsäureanhydrid, das zumindest teilweise als Schmelze vorliegt,
- Einleiten von Schwefeldioxid und Wasser in die Schmelze,
wobei beim Einleiten das Schwefeldioxid bevorzugt als Gas oder als Flüssigkeit und das Wasser bevorzugt als Dampf vorliegen und das Einleiten unter Druck erfolgt und
wobei das Schwefeldioxid und das Wasser mehr bevorzugt vor dem Einleiten miteinander gemischt werden.

Gemäß einer weiteren bevorzugten Ausführungsform der Erfindung ist der Gehalt an Alkalimetallionen in der hergestellten Verbindung der Formel **I** höchstens 500 ppm, bevorzugt höchstens 100 ppm, mehr bevorzugt höchstens 10 ppm.

Gemäß einer weiteren bevorzugten Ausführungsform der Erfindung ist der Gehalt an Alkalimetallionen und Erdalkalimetallionen in der hergestellten Verbindung der Formel **I** insgesamt höchstens 500 ppm, bevorzugt höchstens 100 ppm, mehr bevorzugt höchstens 10 ppm.

Gemäß einer weiteren bevorzugten Ausführungsform der Erfindung ist der Gehalt an Kationen in der hergestellten Verbindung der Formel **I** höchstens 500 ppm, bevorzugt höchstens 100 ppm, mehr bevorzugt höchstens 10 ppm.

Gemäß einer weiteren bevorzugten Ausführungsform der Erfindung wird die erfindungsgemäß hergestellte Verbindung der Formel **I** zum Herstellen eines Absorbers durch eine Vernetzung, insbesondere eine thermische Vernetzung eines Polymermaterials mit der Verbindung der Formel **I,** verwendet.

Gemäß einer weiteren bevorzugten Ausführungsform der Erfindung umfasst das Herstellen des Absorbers zumindest folgende Schritte:
(a) Bereitstellen des Polymermaterials, das optional zumindest teilweise in einem Lösungsmittel gelöst ist, wobei es sich bei dem Lösungsmittel insbesondere um Wasser, bevorzugt um deionisiertes Wasser, handelt;
(b) Mischen des Polymermaterials mit der Verbindung der Formel I, wobei die Verbindung der Formel **I** optional zumindest teilweise in einem Lösungsmittel gelöst sein kann, wobei es sich bei dem Lösungsmittel insbesondere um Wasser, bevorzugt um deionisiertes Wasser, handelt;
(c) optional fortgesetztes mechanisches Behandeln der Mischung, insbesondere Rühren;
(d) optional Trocknen, insbesondere bei erhöhter Temperatur;
(e) Durchführen einer Temperaturbehandlung zum Vernetzen des Polymermaterials mit der Verbindung der Formel **I.**

Gemäß einer weiteren bevorzugten Ausführungsform der Erfindung wird diese Temperaturbehandlung zumindest zeitweise bei einer Temperatur von mindestens 80 °C, bevorzugt mindestens 100 °C, noch bevorzugt mindestens 110 °C durchgeführt.

Gemäß einer weiteren bevorzugten Ausführungsform der Erfindung wird diese Temperaturbehandlung zumindest zeitweise bei einer Temperatur von höchstens 160 °C, bevorzugt höchstens 140 °C, mehr bevorzugt höchstens 130 °C durchgeführt.

Gemäß einer weiteren bevorzugten Ausführungsform der Erfindung wird diese Temperaturbehandlung zumindest zeitweise bei einer Temperatur von etwa 120 °C durchgeführt.

Gemäß einer weiteren bevorzugten Ausführungsform der Erfindung beträgt das Gewichtsverhältnis zwischen der Verbindung der Formel **I** und dem Polymermaterial in dem Verfahren zum Herstellen eines Absorbers oder Superabsorbers mindestens 0,01:1, bevorzugt mindestens 0,02:1, mehr bevorzugt mindestens 0,05:1, noch mehr bevorzugt mindestens 0,1:1

Gemäß einer weiteren bevorzugten Ausführungsform der Erfindung beträgt das Gewichtsverhältnis zwischen der Verbindung der Formel **I** und dem Polymermaterial in dem Verfahren zum Herstellen eines Absorbers oder Superabsorbers höchstens 5:1, bevorzugt höchstens 3:1, mehr bevorzugt höchstens 1:1, noch mehr bevorzugt höchstens 0,5:1, insbesondere bevorzugt höchstens 0,1:1.

Gemäß einer weiteren bevorzugten Ausführungsform der Erfindung wird die erfindungsgemäß hergestellte Verbindung der Formel **I** als Hilfsstoff in einem Verfahren zum elektrochemischen Abscheiden von Metallen und Metalllegierungen und/oder einem galvanotechnischen Verfahren verwendet.

Gemäß einer weiteren bevorzugten Ausführungsform der Erfindung liegt die in einem Verfahren zum elektrochemischen Abscheiden von Metallen und Metalllegierungen und/oder einem galvanotechnischen Verfahren als Hilfsstoff verwendete Verbindung der Formel **I** bevorzugt in einer Elektrolytlösung gelöst vor.

Gemäß einer weiteren bevorzugten Ausführungsform der Erfindung wird die erfindungsgemäß hergestellte Verbindung der Formel **I** zur Quervernetzung eines Polymermaterials zur Herstellung einer quervernetzten Membran oder einer quervernetzten Oberflächenbeschichtung verwendet.

Gemäß einer weiteren bevorzugten Ausführungsform der Erfindung wird die erfindungsgemäß hergestellte Verbindung der Formel **I** zur Quervernetzung eines Polymermaterials zur Herstellung quervernetzter Formkörper verwendet. Besonders bevorzugt handelt es sich dabei bei dem Polymermaterial um ein Polysaccharid, insbesondere um Cellulose I und/oder Cellulose **II**.

Gemäß einer weiteren bevorzugten Ausführungsform der Erfindung wird die erfindungsgemäß hergestellte Verbindung der Formel **I** zur Quervernetzung von Fasern und/oder Kügelchen umfassend Cellulose I und/oder Cellulose II, bevorzugt Fasern und/oder Kügelchen aus Cellulose I und/oder Cellulose II verwendet.

Durch Quervernetzung von Cellulose I und/oder Cellulose II sind gemäß einer weiteren bevorzugten Ausführungsform der Erfindung Membranen aus quervernetzter Cellulose zugänglich.

Gemäß einer weiteren bevorzugten Ausführungsform der Erfindung wird die erfindungsgemäß hergestellte Verbindung der Formel **I** zur Quervernetzung eines Polymermaterials zur Herstellung eines Trägermaterials für Enzyme und/oder Mikroorganismen verwendet. Dabei ist es insbesondere bevorzugt, dass die Enzyme und/oder Mikroorganismen vor oder während der Vernetzungsreaktion der Reaktionsmischung, in der die Vernetzungsreaktion stattfindet, vorhanden sind. Dann ist es möglich, dass die Enzyme und/oder Mikroorganismen zumindest teilweise in und/oder auf dem quervernetzten Polymermaterial immobilisiert sind. Es wird dabei in Betracht gezogen, dass das als Trägermaterial für Enzyme und/oder Mikroorganismen verwendete quervernetzte Polymermaterial gleichzeitig die Eigenschaften eines Absorbers oder Superabsorbers zu verleihen. Dadurch sind beispielsweise Absorber oder Superabsorber zugänglich, die die Enzyme und/oder Mikroorganismen, die für deren späteren biologischen Abbau zuständig sind, bereits in sich tragen.

Das erfindungsgemäße Verfahren zum Herstellen einer aus der aus den Verbindungen mit den Formeln **V, VI** und **VII** bestehenden Gruppe ausgewählten Verbindung 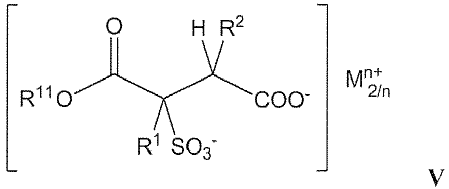 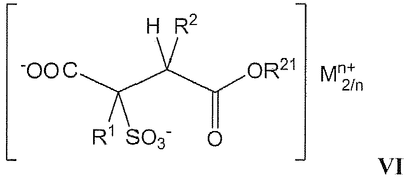 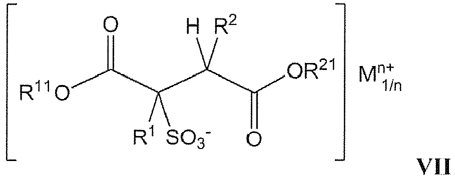 wobei -R¹, -R², -R¹¹ und -R²¹ wie obenstehend definiert sind und wobei Mⁿ⁺ ein n-wertiges Kation, insbesondere ein n-wertiges positives Ion des Metalls M ist, umfasst die Schritte:
- Durchführen eines wie oben beschriebenen Verfahrens zum Herstellen der Verbindung der Formel **I;**
- Umsetzen der Verbindung der Formel I mit einer geeigneten Menge einer Base, die Mⁿ⁺ umfasst, insbesondere mit M(OH)ₙ, und/oder mit einer geeigneten Menge von metallischem M;
- Erhalten der aus der aus den Verbindungen mit den Formeln **V, VI** und **VII** bestehenden Gruppe ausgewählten Verbindung.

Es ist im Rahmen der Erfindung möglich, dass die Verbindung der Formel **I** nach ihrer Herstellung als Komponente einer Mischung vorliegt. Eine solche Mischung wird nachfolgend als "Sulfobernsteinsäurematerial" bezeichnet, auch wenn es sich bei der Verbindung der Formel **I** nicht speziell um Sulfobernsteinsäure, sondern um eine andere davon abgeleitete Verbindung der Formel **I** handelt. In einem Sulfobernsteinsäurematerial können auch mehrere Verbindungen der Formel **I** enthalten sein. Mehrere Verbindungen der Formel **I** können insbesondere dann im Sulfobernsteinsäurematerial enthalten sein, wenn für das erfindungsgemäße Verfahren verschiedene Verbindungen der Formeln **II**, **III** und **IV** als Edukte eingesetzt worden sind. Beispielsweise kann das Sulfobernsteinsäurematerial neben Sulfobernsteinsäure auch 2,3-Dimetylsulfobernsteinsäure enthalten, wenn als Edukte Maleinsäure und 2,3-Dimethylmaleinsäure ((2Z)-2,3-Dimethyl-2-butendisäure) eingesetzt worden sind.

Bei dem Sulfobernsteinsäurematerial handelt es sich beispielsweise um eine Mischung aus mindestens einer Verbindung der Formel **I** mit Wasser, insbesondere um eine wässrige Lösung.

Die Herstellung einer Verbindung der Formel **I,** die frei oder im Wesentlichen frei von Alkalimetallionen ist, oder eines Sulfobernsteinsäurematerials, das frei oder im Wesentlichen frei von Alkalimetallionen ist, ist auf besonders effiziente und ökonomische Weise möglich, wenn dabei im Gegensatz zum genannten Stand der Technik die Verwendung von Alkalimetallsulfitsalzen wie beispielsweise Natriumsulfit, Natriumhydrogensulfit und Natriumdisulfit, Erdalkalimentallsulfitsalzen und Sulfitsalzen anderer Metalle vermieden wird. In diesem Fall werden keine Alkalimetallionen, Erdalkalimetallionen und andere Metallionen eingebracht. Beispielsweise wird Sulfobernsteinsäure in der gewünschten freien Säureform erhalten, ohne dass dafür ein zusätzlicher Ionenaustauschprozess nötig ist.

Eine Verbindung wird als "im Wesentlichen" frei von dem vorstehend definierten Ion bzw. den vorstehend definierten Ionen bezeichnet, wenn die Verunreinigung mit diesem Ion/diesen Ionen so gering ist, dass im Wesentlichen kein störender Einfluss auf den Verlauf einer chemischen Reaktion, für die die Verbindung beispielsweise als Ausgangsstoff eingesetzt wird, gegeben ist. Eine Zusammensetzung wird als "im Wesentlichen" frei von dem vorstehend definierten Ion bzw. den vorstehend definierten Ionen bezeichnet, wenn der Gehalt an diesem Ion/diesen Ionen so gering ist, dass im Wesentlichen kein störender Einfluss auf den Verlauf einer chemischen Reaktion, für die die Zusammensetzung beispielsweise als Ausgangsstoff eingesetzt wird, gegeben ist.

Es hat sich gezeigt, dass ein derartiger störender Einfluss insbesondere dann unterbleibt, wenn der Gehalt an Alkalimetallionen und gegebenenfalls der Gehalt an Erdalkalimetallionen und/oder anderer Kationen in der Verbindung der Formel **I** bzw. einem Sulfobernsteinsäurematerial höchstens 500 ppm, bevorzugt höchstens 100 ppm, mehr bevorzugt höchstens 10 ppm ist.

Eine Verbindung wird als frei von dem vorstehend definierten Ion bzw. den vorstehend definierten Ionen bezeichnet, wenn die Verunreinigung mit diesem Ion/diesen Ionen so gering ist, dass dieses Ion/diese Ionen durch das zum Ionennachweis verwendete Verfahren nicht nachweisbar ist/sind. Eine Zusammensetzung wird als frei von dem vorstehend definierten Ion bzw. den vorstehend definierten Ionen bezeichnet, wenn der Gehalt an diesem Ion/diesen Ionen so gering ist, dass dieses Ion/diese Ionen durch das zum Ionennachweis verwendete Verfahren nicht nachweisbar ist/sind. Die Nachweisgrenze für anorganische Kationen in einer Lösung liegt häufig bei 5 ppm.

Die jeweiligen Angaben des Gehalts an Alkalimetallionen, Erdalkalimetallionen bzw. anderen Kationen in einem Sulfobernsteinsäurematerial werden nachfolgend auf das Gesamtgewicht des Sulfobernsteinsäurematerials bezogen, wobei die Einheit "ppm" als Gewichtskonzentrationsmaß verstanden wird. Folgendes Beispiel soll dies exemplarisch veranschaulichen: Handelt es sich bei dem Sulfobernsteinsäurematerial beispielsweise um eine Sulfobernsteinsäurelösung mit einem Sulfobernsteinsäuregehalt von 70 Gew.-%, so bedeutet ein Natriumionengehalt von 50 ppm, dass 1 kg dieser Sulfobernsteinsäurelösung 50 × 10⁻⁶ kg = 50 mg Natriumionen enthält.

Es ist bevorzugt, dass erfindungsgemäß hergestellte Sulfobernsteinsäure in Form einer wässrigen Lösung mit einem Sulfobernsteinsäuregehalt von 70 Gew.-% vorliegt. Wenn eine derartige Lösung beispielsweise einen Natriumionengehalt von 50 ppm aufweist, bedeutet dies, dass in der Lösung pro Kilogramm Sulfobernsteinsäure 50/0,7 = 71 mg Natriumionen vorhanden sind. Über das Molekulargewicht der Sulfobernsteinsäure von 198,15 g/mol wird berechnet, dass in einer derartigen Lösung pro Mol Sulfobernsteinsäure 71/(1000/198,15) = 14,15 mg (entsprechend 0,61 mmol) Natriumionen enthalten sind.

Die Verbindung der Formel **I** ist bevorzugt frei oder im Wesentlichen frei von Alkalimetallionen. Entsprechendes gilt für ein Sulfobernsteinsäurematerial.

Weiter bevorzugt ist die Verbindung der Formel **I** zusätzlich frei oder im Wesentlichen frei von Erdalkalimetallionen. Weiter bevorzugt ist die Verbindung der Formel **I** zusätzlich frei oder im Wesentlichen frei von weiteren Kationen, insbesondere Metallionen. Entsprechendes gilt für ein Sulfobernsteinsäurematerial. Hydroniumionen bleiben dabei unberücksichtigt.

Die Erfinder haben überraschend gefunden, dass die Herstellung einer Verbindung der Formel **I** durch Reaktion mindestens einer der Verbindungen der Formeln **II**, **III**, **IV** mit Schwefeldioxid bei Anwesenheit von Wasser erfolgen kann.

So haben die Erfinder beispielsweise überraschend gefunden, dass Sulfobernsteinsäure durch Reaktion mindestens einer der aus der aus Maleinsäure, Maleinsäureanhydrid, Fumarsäure und deren Derivaten bestehenden Gruppe ausgewählten Verbindung mit Schwefeldioxid bei Anwesenheit von Wasser hergestellt werden kann.

Unter Derivaten werden dabei insbesondere die Monoester und Diester, die die Maleinsäure und die Fumarsäure mit Alkoholen, bevorzugt mit aliphatischen Alkoholen, mehr bevorzugt mit aliphatischen Alkoholen der Formel CₙH₂ₙ₊₁OH mit n = 1, 2, 3 oder 4 bilden, sowie die Monochloride und Dichloride der Maleinsäure und der Fumarsäure verstanden.

Ein besonderer Vorteil des erfindungsgemäßen Verfahrens zur Herstellung einer Verbindung der Formel **I** bzw. eines Sulfobernsteinsäurematerials unter Verwendung von Schwefeldioxid anstelle von Sulfitsalzen ist die Möglichkeit der völligen Abwesenheit von Alkalimetallen, Erdalkalimetallen und anderen Kationen im Reaktionsprozess und damit einhergehend die Entbehrlichkeit eines zusätzlichen Ionenaustauschschritts.

Es ist möglich, dass im Zuge der Durchführung des erfindungsgemäßen Verfahrens intermediär ein Anhydrid der Verbindung der Formel **I** gebildet wird.

Wenn es sich bei der hergestellten Verbindung der Formel **I** um einen Monoester oder Diester handelt, kann eine Verseifung mittels eines basischen Ionenaustauschers vorgenommen werden, um die freie Säure zu erhalten und dabei die Verwendung einer Lauge, mit der Kationen eingetragen würden, zu vermeiden.

In einer bevorzugten Ausführungsform des erfindungsgemäßen Verfahrens erfolgt die Umsetzung zu mindestens einer Verbindung der Formel **I,** indem Schwefeldioxid zu einer Reaktionsmischung, die mindestens eine der Verbindungen der Formeln **II**, **III** und **IV** enthält, unter Druck eingeleitet wird, wobei das Verhältnis der Stoffmenge des eingesetzten Schwefeldioxids und der Stoffmenge der mindestens einen der Verbindungen der Formeln **II**, **III** und **IV** höchstens 1000, bevorzugt höchstens 500, mehr bevorzugt höchstens 100 und/oder mindestens 1, bevorzugt mindestens 5, mehr bevorzugt mindestens 10 beträgt.

In einer bevorzugten Ausführungsform dieses Verfahrens wird in eine mit Schwefeldioxid gesättigte wässrige Phase Maleinsäure, Maleinsäureanhydrid, Fumarsäure oder eine Mischung aus mindestens zwei dieser Stoffe eingetragen. Maleinsäureanhydrid kann dabei beispielsweise als Feststoff oder in flüssiger Form als Schmelze zugegeben werden. Alternativ oder zusätzlich kann eine unter Verwendung von Maleinsäure, Maleinsäureanhydrid, Fumarsäure oder einer Mischung aus mindestens zwei dieser Stoffe hergestellte wässrige Lösung zugegeben werden. Es wird Schwefeldioxid in das Reaktionsgemisch eingetragen. Der Reaktionsverlauf wird bevorzugt dünnschichtchromatographisch kontrolliert. Bevorzugt wird so lange Schwefeldioxid in das Reaktionsgemisch eingetragen, bis die dünnschichtchromatographische Analyse kein Edukt mehr anzeigt. Der Eintrag von Schwefeldioxid in die Reaktionslösung erfolgt bevorzugt unter Druck. Nach Beendigung der Reaktion wird das Reaktionsgefäß entspannt und die Reaktionslösung an der Atmosphäre erhitzt, um überschüssiges gelöstes Schwefeldioxid auszutreiben. Die erhaltene wässrige Lösung von Sulfobernsteinsäure ist völlig frei von jeglichen Alkalimetallionen, Erdalkalimetallionen und anderen Kationen.

Gemäß einer weiteren bevorzugten Ausführungsform dieses Verfahrens werden gasförmiges Schwefeldioxid und Wasserdampf unter Druck in eine Schmelze von Maleinsäureanhydrid eingetragen, um das Maleinsäureanhydrid zur Sulfobernsteinsäure umzusetzen.

Gemäß einem Anwendungsbeispiel der Erfindung wird die erfindungsgemäß hergestellte Verbindung der Formel **I** als Quervernetzer bei der Herstellung von Absorbern verwendet. Die Verbindung der Formel **I** kann dabei in Form eines Sulfobernsteinsäurematerials verwendet werden. Unter Absorbern werden Polymermaterialien mit einer Kapazität zur Anlagerung bzw. Absorption von Flüssigkeiten, insbesondere polaren Flüssigkeiten und vor allem wässrige Flüssigkeiten, verstanden. Absorber sind somit in der Lage, Flüssigkeiten aufzunehmen und zurückzuhalten. Bei einer polaren Flüssigkeit handelt es sich insbesondere um Wasser und wässrige Flüssigkeiten, beispielsweise wässrige Lösungen oder Dispersionen organischer und/oder anorganischer Substanzen. Im Falle von Superabsorbern ist die Menge der Flüssigkeit, die aufgenommenen und zurückgehaltenen werden kann, groß, in der Regel ein Vielfaches des Eigengewichts des absorbierenden Kunststoffmaterials.

Bei Absorbern handelt es sich insbesondere um ein poröses und/oder faserartiges und quervernetztes Polymer. Der Begriff Polymer umfasst dabei Homopolymere, alle Arten von Co-Polymeren und Polyblends. Absorber und Superabsorber sind grundsätzlich bekannt. Beispielhaft wird auf F.L. Buchholz und AT. Graham, "Modern Superabsorbent Polymer Technology", Wiley-VCH, 1998, Seiten 69 bis 117 verwiesen. Absorber und insbesondere Superabsorber werden beispielsweise in Babywindeln, Damenbinden, Inkontinenzprodukten und Verbandmaterial zum Aufnehmen von Körperausscheidungen, beispielsweise Harn, und anderen Körperflüssigkeiten, beispielsweise Blut, verwendet. Ferner werden Absorber und insbesondere Superabsorber beispielsweise zur Herstellung gelbildender Löschmittel zur Brandbekämpfung, zur Herstellung von Gelbetten (spezielle Art von Wasserbetten) und zur Erhöhung der Wasserspeicherkapazität von Böden zum Kultivieren von Nutz- und Zierpflanzen verwendet.

Die im Stand der Technik bekannten Absorber und Superabsorber weisen häufig kein definiertes Quellverhalten auf, d.h. es ist beispielsweise nicht möglich, das absorbierende Grundmaterial - insbesondere bedingt durch dessen Herstellung - so zu präparieren, dass eine definierte oder gewünschte Menge der zu absorbierenden Flüssigkeit von einer vorgegebenen Menge des Absorbers oder Superabsorbern aufgenommen wird. Folglich kann die Saugfähigkeit von Produkten, beispielsweise Hygieneartikeln, die mit herkömmlichen Absorbern oder Superabsorbern hergestellt sind, nicht oder nicht genau reproduzierbar eingestellt werden. Um sicherzustellen, dass derartige Produkte trotz der schlechten Reproduzierbarkeit der Saugfähigkeit ein gewünschtes Mindestmaß an Saugfähigkeit aufweisen, wird herkömmlich häufig ein großer Überschuss an Absorbern oder Superabsorbern eingesetzt, was aus ökologischen und ökonomischen Gründen nachteilig ist. Ferner ist es herkömmlich nur unzureichend oder nicht zufriedenstellend möglich, zu bestimmen, wie sich das Volumen eines Absorbers oder Superabsorbers bei der Flüssigkeitsaufnahme verändert und mit welcher Geschwindigkeit die Flüssigkeitsaufnahme erfolgt. Folglich ist im Falle von unter Verwendung herkömmlicher Absorber und Superabsorber hergestellten Produkten nicht nur die Saugfähigkeit schlecht reproduzierbar, sondern es ist bei solchen Produkten auch nicht in reproduzierbarer Weise vorherzubestimmen, ob die Aufnahme von Flüssigkeit schnell oder langsam erfolgt und wie stark dabei die Volumenzunahme ist. Aufgrund der vorteilhaften Produkteigenschaften der erfindungsgemäß hergestellten Verbindung der Formel **I** werden diese herkömmlichen Nachteile vermieden.

Die im Stand der Technik bekannten Absorber und Superabsorber sind außerdem nur zum Teil biologisch abbaubar. Ein rascher und nach Möglichkeit vollständiger biologischer Abbau von Absorbern und Superabsorbern ist jedoch im Hinblick auf den Umweltschutz und die Verbraucherakzeptanz vorteilhaft. Die erfindungsgemäß hergestellte Verbindung der Formel **I** kann einem damit hergestellten vernetzten Produkt biologische Abbaubarkeit verleihen.

Gemäß dem Anwendungsbeispiel wird ein Absorber, insbesondere ein Superabsorber durch die Vernetzung eines Polymermaterials mit einer Verbindung der Formel **I,** die beispielsweise als Sulfobernsteinsäurematerial eingesetzt wird, erhalten.

Vorzugsweise enthält das Polymermaterial, welches erfindungsgemäß vernetzt wird, Hydroxygruppen und/oder Aminogruppen.

Vorzugsweise enthält das Polymermaterial, welches erfindungsgemäß vernetzt wird, ionische Gruppen, die dem Absorber eine - vorzugsweise große oder sogar sehr große - Kapazität zur Absorption/Aufnahme/Bindung von Wasser und anderen polaren Flüssigkeiten verleihen. Bei diesen Gruppen handelt es sich beispielsweise um Carboxylatguppen (-COO⁻). Entsprechend enthält das zu vernetzende Polymermaterial beispielsweise Hydroxygruppen und/oder Aminogruppen und optional zusätzlich Carboxygruppen und/oder Natriumcarboxylatgruppen und/oder Kaliumcarboxylatgruppen.

Im Rahmen der Erfindung ist es bevorzugt, dass das Polymermaterial ein Polymer umfasst, das aus der aus Polyvinylalkohol, Cellulose I, Cellulose II, Chitosan, Stärke, Alginaten, Carrageenen, Dextranen, Cyclodextrinen, Copolymeren der Acrylsäure und Natriumacrylaten bestehenden Gruppe ausgewählt ist.

Cellulose I, Cellulose II, Chitosan, Stärke, Alginate, Dextrane, Cyclodextrine und Carrageenen sind dabei besonders leicht biologisch abbaubar. Daher können unter Verwendung dieser Polymere Absorber oder Superabsorber hergestellt werden, die besonders leicht biologisch abbaubar sind.

Unter Verwendung Copolymere der Acrylsäure und Natriumacrylate lassen sich durch Vernetzung mit einem Sulfobernsteinsäurematerial Absorber oder Superabsorber herstellen, die eine besonders hohe Absorptionsfähigkeit aufweisen, d.h. die bezogen auf ihr Eigengewicht eine besonders große Menge an Wasser und anderen polaren Flüssigkeiten aufnehmen und zurückhalten können.

Es wurde überraschend gefunden, dass die Vernetzungsreaktion des Polymermaterials nicht gestört wird, wenn darauf geachtet wird, dass die eingesetzte Verbindung der Formel **I** frei oder im Wesentlichen frei von Alkalimetallionen, insbesondere frei oder im Wesentlichen frei von Alkalimetallionen und Erdalkalimetallionen sowie anderen Kationen ist, so dass der Absorber gut definierbar und einstellbar herzustellen ist, während gleichzeitig das Quellverhalten in einer herkömmlich weder in Betracht gezogenen noch zu erwartenden Weise eingestellt werden kann. Außerdem können die genannten Ionen dann keinen störenden Einfluss auf nachfolgende Reaktionen der Verbindung der Formel **I,** insbesondere auf deren Vernetzungsreaktionen haben. Es hat sich gezeigt, dass die Vernetzungsreaktion durch die Einlagerung von Alkalimetallsalzen oder Salzen, die von anderen Kationen mit Anionen gebildet werden, beispielsweise die Einlagerung von Natriumsalzen der der Sauerstoffsäuren des Schwefels (beispielsweise Natriumsulfat, Natriumhydrogensulfat, Natriumsulfit, Natriumhydrogensulfit) gestört wird. Erfindungsgemäß wird die Einlagerung solcher Salze vermieden.

Durch die Verwendung von erfindungsgemäß hergestellter Verbindung der Formel **I** ist es beispielsweise möglich, dass durch deren Zugabe zu einem Polymermaterial der Ionengehalt dieses Polymermaterials nicht oder nur unwesentlich durch den Eintrag von zusätzlichen Alkalimetallionen und gegebenenfalls Erdalkalimetallionen und/oder anderen Kationen verändert wird. Folglich ist es etwa möglich, eine Beeinflussung des Quellverhaltens eines unter Verwendung einer Verbindung der Formel **I** hergestellten Absorbers oder Superabsorbers, die auf den Eintrag zusätzlicher Alkalimetallionen zurückzuführen ist, zu verringern oder überhaupt zu vermeiden und die Eigenschaften des Absorbers oder Superabsorbers, insbesondere die Absorptionsfähigkeit und die Kinetik des Quellvorgangs, genau einstellen zu können.

Ein weiterer Vorteil der Verwendung der erfindungsgemäß hergestellten Verbindung der Formel **I** als Quervernetzer und/oder Oberflächenvernetzer zur Herstellung von Absorbern ergibt sich im Falle von Absorbern und Superabsorbern, die durch Vernetzung eines Polymermaterials, das neben -OH Gruppen auch Carboxygruppen -COOH und Carboxylatgruppen -COO⁻ enthält, erhältlich sind. Die Anwesenheit von Carboxylatgruppen -COO⁻ im Polymer bedingt die Anwesenheit von Kationen, bei denen es sich insbesondere um Alkalimetallionen handelt, zum Ladungsausgleich. Das Quellverhalten eines durch Quervernetzung eines derartigen Polymermaterials unter Verwendung der erfindungsgemäß hergestellten Verbindung der Formel **I** hergestellten Absorbers wird erfindungsgemäß praktisch nur noch durch die gut definierbare Menge an ionischen Bestandteilen, welche in der Polymermatrix gebunden sind, bestimmt und nicht durch einen unkontrollierbaren Eintrag durch eine mit Ionen verunreinigte Verbindung der Formel **I** beeinträchtigt. Für das Quellverhalten maßgeblich ist somit allein das Verhältnis zwischen der Anzahl an Carboxygruppen (-COOH) einerseits und der Anzahl an Carboxylatgruppen, die die Anwesenheit beispielsweise eines Alkalimetallions X⁺ zum Ladungsausgleich bedingen, andererseits (-COO⁻X⁺), d.h. das Verhältnis -COO⁻X⁺ : -COOH im Polymermaterial. Um die Gesamtmenge an einwertigen Alkalimetallionen beim Herstellen von Absorbern oder Superabsorbern genau einstellen zu können und Absorber oder Superabsorber hoher Qualität und hoher Reproduzierbarkeit herzustellen, ist es nötig und erfindungsgemäß möglich, deren Gehalt in den bei der Herstellung verwendeten Ausgangsstoffen zu kennen und zu definieren. Erfindungsgemäß können somit Polymerketten mit definierter Ionenkonzentration eingesetzt und mit Quervernetzern behandelt werden, um initiale Körnchen des Absorbers oder Superabsorbers zu formen, d.h. um aus den meist wasserlöslichen Polymeren durch Quervernetzung einen wasserunlöslichen Absorber oder Superabsorber mit körniger Struktur herzustellen. Optional können die initialen Körnchen durch eine Oberflächenvernetzung, d.h. eine weitere Vernetzung in oberflächennahen Bereichen des Körnchens, nachbehandelt werden. Durch Auswahl und Einstellung des Quervernetzers gemäß der Erfindung, der den Ionengehalt des hergestellten Absorbers oder Superabsorbers nicht oder nur in geringem Maße verändert, ist es auf einfache Weise möglich, die Gesamtmenge an einwertigen Ionen genau einzustellen und Absorber oder Superabsorber hoher Qualität und hoher Reproduzierbarkeit bereitzustellen.

Darüber hinaus ist bei einem hohen Gehalt an Alkalimetall- und/oder anderen Kationen die Quervernetzungsreaktion gestört. Beispielsweise kann bei Anwesenheit derartiger Ionen die Geschwindigkeit der Quervernetzungsreaktion beträchtlich herabgesetzt sein. Durch die Verwendung der erfindungsgemäß hergestellten Verbindung der Formel **I** ist es möglich, zu verhindern, dass der Gehalt an Ionen ein für den gewünschten Verlauf der Quervernetzungsreaktion akzeptables Maß überschreitet. Beispielsweise können zwei- oder höherwertige Ionen mit dem Polymermaterial ein dreidimensionales ionisches Gelnetzwerk ausbilden, wodurch die gewünschte kovalente Vernetzung unterdrückt wird. Zum Beispiel gelieren Alginate bei Anwesenheit von Calciumionen. Bei Trocknung kollabieren diese Netzwerke jedoch irreversibel und zeigen nur mehr ein sehr geringes bzw. kein Quellverhalten.

Durch den Einsatz von alkalifreiem oder im Wesentlichen alkalifreiem Sulfobernsteinsäurematerial konnte ein Quervernetzer gefunden werden, der nicht nur biologisch abbaubar, sondern auch für die Herstellung eines Absorbers oder Superabsorbers mit definiertem Quellverhalten geeignet ist. Dies wird durch das erfindungsgemäße Verfahren zur Herstellung von Sulfobernsteinsäurematerial unter Einsatz von Schwefeldioxid ermöglicht.

Auf diese Weise hergestellte Absorber werden beispielsweise in einem der aus der aus Schäumen, Formkörpern, Fasern, Folien, Filmen, Kabeln, Dichtungsmaterialien, flüssigkeitsaufnehmenden Hygieneartikeln, Trägern für pflanzen- und pilzwachstumsregulierende Mittel, Wasserspeichergranulaten für die Verwendung im Agrarsektor, gelbildenden Löschmittel, Verpackungszusätzen, Baustoffen, Damenhygieneprodukten, Babyhygieneprodukten, Tierhygieneprodukten, Wundauflagen, Erwachseneninkontinenzprodukten, für chromatographische Trennungen verwendbaren Absorbermaterialien, insbesondere Säulenmaterialien, Trägermaterialien für Enzyme und/oder Mikroorganismen, für Membrantrennungen verwendbaren Materialien sowie im Körperpflege- und/oder Kosmetikbereich einsetzbaren Absorbermaterialien bestehenden Gruppe ausgewählten Produkt verwendet.

Gemäß einem konkreten Verwendungsbeispiel der Erfindung wird die erfindungsgemäß hergestellte Verbindung der Formel **I** in Form einer wässrigen Sulfobernsteinsäurelösung als Quervernetzer bei der Herstellung von Absorbern verwendet.

Gemäß einem weiteren Verwendungsbeispiel der Erfindung wird die erfindungsgemäß hergestellte Verbindung der Formel **I** als Hilfsstoff bei einen elektrochemischen Prozess verwendet. Dabei wird die Verbindung der Formel **I** bevorzugt in einer für den elektrochemischen Prozess verwendeten Elektrolytlösung gelöst. Alternativ oder zusätzlich kann die Verbindung der Formel **I** als Leitelektrolyt eingesetzt werden. Bei dem elektrochemischen Prozess kann es sich beispielsweise um eine elektrochemische Abscheidung eines Metalls oder einer Metalllegierung, um ein Elektroplattieren, einen anderen galvanotechnischen Prozess oder einen Elektrolyseprozess handeln. Vorteilhaft lässt sich beim Elektroplattieren durch Zusatz der erfindungsgemäß hergestellten Verbindung der Formel **I** zur Elektrolytlösung die Qualität der elektrochemisch abgeschiedenen Metallschichten verbessern, ohne dass dadurch Alkalimetallionen, Erdalkalimetallionen oder andere Ionen, die den elektrochemischen Prozess stören, eingetragen werden. Eine Verbesserung der Qualität der abgeschiedenen Metallschichten bedeutet dabei insbesondere, dass Metallschichten abgeschieden werden, die gleichmäßiger und homogener sind sowie weniger Löcher aufweisen als in der Abwesenheit des Hilfsstoffs abgeschiedene Metallschichten.

Gemäß einem konkreten Verwendungsbeispiel der Erfindung wird die erfindungsgemäß hergestellte Verbindung der Formel **I** in Form von Sulfobernsteinsäure, die in der bei einem elektrochemischen Prozess verwendeten Elektrolytlösung gelöst ist, verwendet.

Gemäß einem weiteren Verwendungsbeispiel der Erfindung wird die erfindungsgemäß hergestellte Verbindung der Formel **I** als Quervernetzer bei der Herstellung einer Membran oder einer Beschichtung ausgehend von einem Polymermaterial verwendet. Dazu wird das Polymermaterial mit der Verbindung der Formel **I** gemischt. Bevorzugt werden das Polymermaterial und die Verbindung der Formel **I** dabei in einem Lösungsmittel gelöst. Die Mischung wird auf eine Oberfläche aufgebracht. Optional wird die Mischung getrocknet. Anschließend wird die auf die Fläche aufgebrachte Mischung einer Temperaturbehandlung unterzogen, um die Quervernetzung des Polymermaterials durchzuführen. Bei dem quervernetzten Polymermaterial handelt es sich um eine Beschichtung der Oberfläche. Wird das quervernetzte Polymermaterial von der Oberfläche abgelöst, wird eine Membran erhalten. Dabei ist es durch die Verwendung der erfindungsgemäß hergestellten Verbindung der Formel **I** möglich, die Quervernetzungsreaktion genau zu kontrollieren, wodurch für die jeweilige Anwendung eine beispielsweise hinsichtlich der mechanischen Eigenschaften und der Porosität genau eingestellte Membran erhalten werden kann.

Durch die Verwendung der erfindungsgemäß hergestellten Verbindung der Formel **I** wird vermieden, dass Alkalimetallionen, Erdalkalimetallionen oder andere Kationen die Quervernetzungsreaktion stören.

In analoger und gleichermaßen vorteilhafter Weise sind Formkörper zugänglich. Dazu wird die Mischung vor der Durchführung der Quervernetzung nicht auf einer Oberfläche verteilt, sondern in eine Form eingebracht. Darüber hinaus ist auch die Quervernetzung bzw. Oberflächenvernetzung von bereits vorhandenen Formkörpern möglich.

In einem konkreten Verwendungsbeispiel der Erfindung wird zu einer wässrigen Polyvinylalkohollösung mit einem PVA-Gehalt von 10 Gew.-% Sulfobernsteinsäure hinzugefügt. Die Menge an Sulfobernsteinsäure wird dabei so gewählt, dass zwischen 5 und 30 Gew.-% Sulfobernsteinsäure in der Mischung enthalten sind. Die Mischung wird 24 Stunden lang gerührt. Die erhaltene homogene Lösung wird auf eine Acrylglasplatte gegossen und darauf verteilt. Nach dem Trocknen bei 60 °C im Konvektionsofen wird eine Temperaturbehandlung bei einer Temperatur zwischen 120 und 130 °C durchgeführt. Das quervernetzte PVA-Material wird von der Acrylglasplatte gelöst, um eine Membran zu erhalten.

Gemäß einem weiteren Verwendungsbeispiel wird die erfindungsgemäß hergestellte Verbindung der Formel **I** als Quervernetzer zum Quervernetzen von Cellulose I und/oder Cellulose II verwendet, um daraus Formkörper herzustellen oder um bereits auf verschiedene Weise geformte Formkörper gegen Druck oder Kollabieren bei Trocknung zu stabilisieren bzw. hinsichtlich ihres mechanischen Verhaltens und ihrer Porosität genau zu bestimmen. Diese Formkörper sind in Folge der Quervernetzung formstabil. Je nach Wahl des Mengenverhältnisses zwischen der eingesetzten Verbindung der Formel **I** und der eingesetzten Cellulose I und/oder Cellulose II kann die Quervernetzung dabei stärker oder weniger stark ausgeprägt sein, wodurch sich Formkörper mit unterschiedlicher Härte und Festigkeit ergeben. Die Cellulose I und/oder Cellulose II kann dabei beispielsweise in Form von faserförmigem oder partikelförmigem Ausgangsmaterial eingesetzt werden.

Gemäß einem konkreten Verwendungsbeispiel der Erfindung wird die erfindungsgemäß hergestellte Verbindung der Formel **I** in Form einer wässrigen Sulfobernsteinsäurelösung verwendet, um Fasern umfassend Cellulose I und/oder Cellulose II, insbesondere Fasern aus Cellulose I und/oder Cellulose II querzuvernetzen.

Gemäß einer Ausführungsform der Erfindung wird die erfindungsgemäß hergestellte Verbindung der Formel **I** mit einer geeigneten Menge einer Base, die Mⁿ⁺ umfasst, insbesondere mit einer geeigneten Menge an M(OH)ₙ, und/oder mit einer geeigneten Menge von metallischem M umgesetzt, um eine aus der aus den Verbindungen mit den Formeln **V, VI** und **VII** bestehenden Gruppe ausgewählten Verbindung herzustellen, 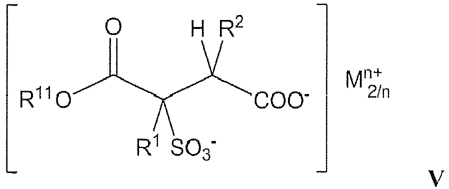 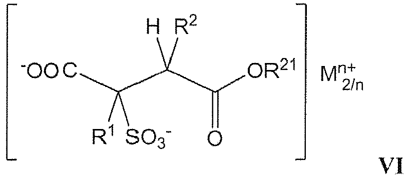 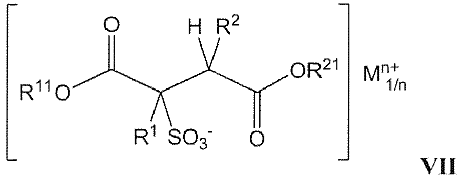 -R¹, -R², -R¹¹ und -R²¹ sind dabei wie obenstehend definiert. Dabei sind -R¹, -R² insbesondere bevorzugt beide Wasserstoff. Mⁿ⁺ ein n-wertiges Kation, mehr bevorzugt ein n-wertiges positives Ion des Metalls M.

Die Base umfasst Mⁿ⁺. Besonders bevorzugt wird als Base eine geeignete Menge M(OH)ₙ verwendet.

Es ist dabei erfindungsgemäß durch die Auswahl der Base und/oder des Metalls M möglich, das n-wertige Kation gezielt und an die jeweilige Anwendung der Verbindung der Formel **V, VI** oder **VII** angepasst auszuwählen und hinsichtlich der eingesetzten Menge genau zu definieren, vorzugsweise ohne Überschuss einzusetzen. Beispielsweise ist es möglich, durch den Einsatz von Natriumhydroxid als der zur Umsetzung verwendeten Base gezielt Na⁺ als Kation M⁺ auszuwählen.

Gemäß einem Verwendungsbeispiel der Erfindung werden die Verbindungen der Formeln **V, VI** und **VII** jeweils als grenzflächenaktive Substanz, insbesondere als Tensid, verwendet.

Die nachfolgend beschriebenen Beispiele dienen der Veranschaulichung vorliegender Erfindung und beschränken daher den Umfang vorliegender Erfindung in keiner Weise. Für den Fachmann ist es im Rahmen der gesamten Offenbarung offensichtlich, dass die nachfolgend beschriebenen Beispiele abgewandelt und modifiziert werden können.

Bei den Beispielen 1 bis 3 handelt es sich um Beispiele der Herstellung von Sulfobernsteinsäure in Form einer Sulfobernsteinsäurelösung, welche frei oder im Wesentlichen frei von Natriumionen und anderen Kationen ist, unter Verwendung von Schwefeldioxid.

Bei Beispiel 4 handelt es sich um ein Beispiel für die erfindungsgemäße Verwendung von Sulfobernsteinsäure zur Herstellung eines Absorbers oder Superabsorbers. Dabei kommt die Sulfobernsteinsäure in Form einer Sulfobernsteinsäurelösung, welche frei oder im Wesentlichen frei von Natriumionen und anderen Kationen ist, zu Einsatz.

### Beispiel 1:

Ein Reaktionsgefäß wurde mit 100 ml Wasser befüllt und Schwefeldioxid bei Raumtemperatur bis zur Sättigung eingeleitet. Danach wurde das Reaktionsgefäß verschlossen und der Reaktionsansatz auf 150°C erhitzt. 10 g Maleinsäure wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 117 g einer klaren wässrigen Lösung von Sulfobernsteinsäure mit einem Sulfobernsteinsäuregehalt von 14,6 Gew.-%. Diese Lösung wurde daraufhin auf eine Lösung mit einem Sulfobernsteinsäuregehalt von 70 Gew.-% am Vakuum einrotiert. Daraufhin wurde mittels ICP-AAS der Natriumgehalt dieser Lösung ermittelt. Er betrug weniger als 5 ppm.

### Beispiel 2:

Ein Reaktionsgefäß wurde mit 130 ml Wasser befüllt und Schwefeldioxid bei Raumtemperatur bis zur Sättigung eingeleitet. Danach wurde das Reaktionsgefäß verschlossen und der Reaktionsansatz auf 170°C erhitzt. 10 g Maleinsäureanhydrid wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 147 g einer klaren wässrigen Lösung von Sulfobernsteinsäure mit einem Sulfobernsteinsäuregehalt von 11,6 Gew.-%. Nach dem Einrotieren dieser Lösung auf eine Lösung mit einem Sulfobernsteinsäuregehalt von 70 Gew.-% am Vakuum wurde der Natriumgehalt dieser Lösung mittels ICP-AAS bestimmt. Er betrug weniger als 5 ppm.

### Beispiel 3:

Ein Reaktionsgefäß wurde mit 500 ml Wasser befüllt und Schwefeldioxid bei Raumtemperatur bis zur Sättigung eingeleitet. Danach wurde das Reaktionsgefäß verschlossen und der Reaktionsansatz auf 180°C erhitzt. 50 g geschmolzener Maleinsäureanhydrid wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 600 g einer klaren wässrigen Lösung von Sulfobernsteinsäure mit einem Sulfobernsteinsäuregehalt von 16,8 Gew.-%. Diese Lösung wurde auf eine Lösung mit einem Sulfobernsteinsäuregehalt von 70 Gew.-% am Vakuum einrotiert. Daraufhin wurde mittels ICP-AAS Natriumgehalt dieser Lösung ermittelt. Er betrug weniger als 5 ppm.

### Beispiel 4:

Polyvinylalkohol (PVA) wurde in deionisiertem Wasser bei 90 °C unter Rühren gelöst, wobei für mindestens 6 Stunden gerührt wurde, um eine Lösung mit mindestens 10 Gew.-% PVA zu erhalten. Diese Lösung wurde mit verschiedenen Mengen an Sulfobernsteinsäurelösung (nachfolgend auch als SSA bezeichnet), die in einem der Beispiele 1 bis 3 erhalten worden war und die daher frei von Alkalimetallionen war, vermischt und die dabei erhaltenen Mischungen A bis F jeweils für 24 Stunden gerührt. Die Mischungen A bis F unterscheiden sich dabei hinsichtlich des Gewichtsverhältnisses der in ihnen enthaltenen Sulfobernsteinsäure zu dem in ihnen enthaltenen Polyvinylalkohol (Gewichtsverhältnis SSA:PVA). Das Gewichtsverhältnis SSA:PVA ist für die Mischungen A bis F in Tabelle 1 angegeben.

**Tabelle 1:**

| **Mischung** | **Gewichtsverhältnis SSA:PVA** |
|---|---|
| A | 0,1:1 |
| B | 0,2:1 |
| C | 0,3:1 |
| D | 0,4:1 |
| E | 0,5:1 |
| F | 3:1 |

Nach dem Rühren wurden diese Lösungen bei 60 °C am Vakuum einrotiert bis kein Wasser mehr abzuziehen war. Diese Masse wurde daraufhin jeweils in eine Kristallisierschale gegossen und im Trockenschrank bei je 60 °C für 3 Stunden getrocknet. Die Schichthöhe nach dem Trocknen betrug dabei etwa 5 mm. Danach erfolgte eine Temperaturbehandlung bei 120 °C für 2 Stunden, wodurch sich durch Quervernetzung ein in wässrigen Flüssigkeiten unlösliches Polymermaterial bildete. Der mittels ICP-AAS bestimmte Natriumgehalt des so hergestellten Polymermaterials betrug weniger als 5 ppm.

Das Quellverhalten des im Beispiel 7 hergestellten vernetzten Polymermaterials wurde wie folgt ermittelt:
Eine Probe mit bekannter Masse wurde in ein Filtersäckchen mit bekannter Masse (konkret in einen Teebeutel) gegeben und für 30 min in vollentsalztes Wasser eingetaucht. Danach war die Probe vollständig gequollen, d.h. sie nahm bei weiterem Versetzen mit Wasser keine größere Menge an Wasser mehr auf. Danach tropfte die Probe 30 min lang ab und wurde anschließend gewogen, um die gesamte Wasseraufnahme durch die Probe und den Teebeutel zu ermitteln. Von dieser gesamten Wasseraufnahme wurde die zuvor ermittelte Wasseraufnahme eines Teebeutels abgezogen, um die Wasseraufnahme der Probe zu berechnen.

Ein einer definierten Einwaage entsprechender kleiner Teil der so behandelten Probe wurde in einer DSC-Messeinrichtung eingefroren. Dabei gefror das freie Wasser, während das gebundene Wasser nicht gefror. Durch anschließendes Ermitteln der Schmelzenergie des gefrorenen Wassers mittels DSC (Mettler-Toledo DSC 3) konnte die Menge des freien Wassers ermittelt werden. Die Menge an gebundenem Wasser wurde berechnet, indem die Differenz zwischen der dem für die DSC-Messung verwendeten Teil der Probe entsprechenden Wasseraufnahme und der mittels DSC ermittelten Menge an freiem Wasser berechnet wurde.

In Reaktionsschema 1 ist die Quervernetzung von PVA mit Sulfobernsteinsäure schematisch dargestellt.

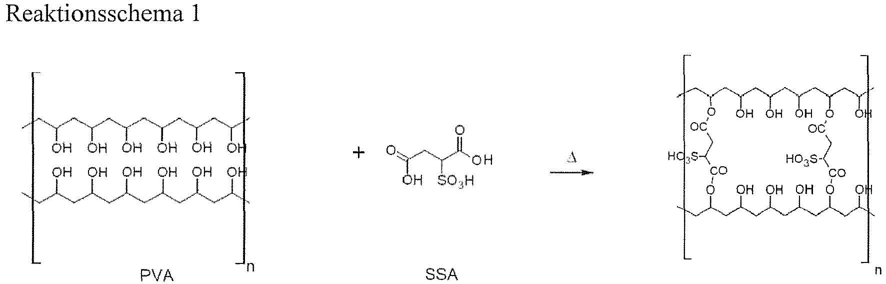

In Tabelle 2 sind für die Mischungen A bis E jeweils die Wasseraufnahme der Probe, die Menge an gebundenem Wasser sowie die Menge an freiem Wasser angegeben. Diese drei Parameter sind dabei jeweils auf die Masse des vernetzten Polymermaterials vor dem Versetzen mit Wasser, d.h. auf die Trockemnasse der Probe, bezogen.

**Tabelle 2**

| **Mischung** | **Gewichtsverhältnis SSA:PVA** | **Wasseraufnahme [Gew.-%]** | **Gebundenes Wasser [Gew.-%]** | **Freies Wasser** [**Gew**.-%] |
|---|---|---|---|---|
| A | 0,1:1 | 193% | 155,4% | 37,6% |
| B | 0,2:1 | 147% | 118,4% | 28,6% |
| C | 0,3:1 | 124% | 99,8% | 24,2% |
| D | 0,4:1 | 91% | 73,3% | 17,7% |
| E | 0,5:1 | 78% | 62,8% | 15,2% |
| F | 3:1 | 43% | 34,6% | 8,4% |

## Patentansprüche

1. Verfahren zum Herstellen einer Verbindung der Formel **I** 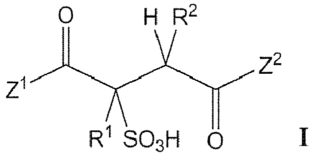 durch Umsetzen mindestens einer aus der aus den Verbindungen mit den Formeln **II**, **III** und **IV** bestehenden Gruppe ausgewählten Verbindung mit Schwefeldioxid in Gegenwart von Wasser, 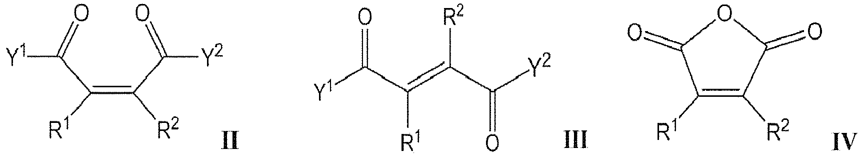
wobei -R¹ und R² unabhängig voneinander aus der aus -H und Alkyl bestehenden Gruppe ausgewählt werden,
wobei -Z¹ aus der aus -OH, -OR¹¹, -NH₂, -NHR¹², -NHR¹²R¹³ und -Cl bestehenden Gruppe ausgewählt wird,
wobei -Z² aus der aus -OH, -OR²¹, -NH₂, -NHR²², -NHR²²R²³ und -Cl bestehenden Gruppe ausgewählt wird,
wobei -Y¹ aus der aus -OH, -OR¹¹, -NH₂, -NHR¹², -NHR¹²R¹³ und -Cl bestehenden Gruppe ausgewählt wird,
wobei -Y² aus der aus -OH, -OR²¹, -NH₂, -NHR²², -NHR²²R²³ und -Cl bestehenden Gruppe ausgewählt wird,
wobei -R¹¹, -R¹², -R¹³, -R²¹, -R²², -R²³ unabhängig voneinander aus Alkylresten ausgewählt werden.

2. Verfahren gemäß Anspruch 1,
wobei R¹ und R² unabhängig voneinander aus der aus -H, Methyl, Ethyl, *n*-Propyl, iso-Propyl, *n*-Butyl, *iso*-Butyl, *sec*-Butyl,*tert*-Butyl und 2-Ethylhexyl bestehenden Gruppe bestehenden Gruppe ausgewählt werden, wobei bevorzugt -R¹ = -R² = -H, und/oder wobei -R¹¹, -R¹², -R¹³, -R²¹, -R²², -R²³ unabhängig voneinander aus der aus Methyl, Ethyl, *n*-Propyl, *iso*-Propyl, *n*-Butyl, *iso*-Butyl, *sec*-Butyl, *tert*-Butyl und 2-Ethylhexyl bestehenden Gruppe ausgewählt werden.

3. Verfahren gemäß einem der vorstehenden Ansprüche,
wobei -Y¹ aus der aus -OH und -OR¹¹ bestehenden Gruppe ausgewählt wird und bevorzugt -OH ist und/oder
wobei -Y² aus der aus -OH und -OR²¹ bestehenden Gruppe ausgewählt wird und bevorzugt -OH ist,
wobei -R¹¹ und -R¹² jeweils wie in Anspruch 1 definiert sind.

4. Verfahren gemäß einem der vorstehenden Ansprüche,
wobei die Verbindung mit der Formel **I** nach ihrer Herstellung in Form einer wässrigen Lösung vorliegt.

5. Verfahren gemäß einem der vorgenannten Ansprüche,
wobei die Temperatur mindestens 0 °C, bevorzugt mindestens 10 °C, noch mehr bevorzugt mindestens 20 °C beträgt und/oder
wobei bevorzugt höchstens 250 °C, mehr bevorzugt höchstens 220 °C, noch mehr bevorzugt höchstens 190 °C beträgt.

6. Verfahren gemäß einem der vorstehenden Ansprüche, umfassend die Schritte:
- Bereitstellen von Wasser;
- optional Lösen von Schwefeldioxid im Wasser;
- Beimengen mindestens einer aus der aus den Verbindungen mit den Formeln **II, III** und **IV** bestehenden Gruppe ausgewählten Verbindung;
- wobei -R¹, -R², -Y¹ und -Y² wie in Anspruch 1 definiert sind;
- Einleiten von Schwefeldioxid.

7. Verfahren gemäß Anspruch 6,
wobei das Schwefeldioxid in Form von Schwefeldioxidgas und/oder flüssigem Schwefeldioxid eingeleitet wird,
wobei das Einleiten bevorzugt unter Überdruck erfolgt,
wobei das Verhältnis der Stoffmenge des eingesetzten Schwefeldioxids und der Stoffmenge der eingesetzten aus den Verbindungen mit den Formeln **II**, **III** und **IV** bestehenden Gruppe ausgewählten Verbindung bevorzugt höchstens 1000, mehr bevorzugt höchstens 500, noch mehr bevorzugt höchstens 100 und/oder bevorzugt mindestens 1, mehr bevorzugt mindestens 5, noch mehr bevorzugt mindestens 10 ist.

8. Verfahren gemäß einem der Ansprüche 6 oder 7,
wobei die mindestens eine aus der aus den Verbindungen mit den Formeln **II**, **III** und **IV** bestehenden Gruppe ausgewählte Verbindung zumindest teilweise in Form einer wässrigen Lösung beigemengt wird.

9. Verfahren gemäß einem der Ansprüche 6 bis 8,
wobei die mindestens eine aus der aus den Verbindungen mit den Formeln **II**, **III** und **IV** bestehenden Gruppe ausgewählte Verbindung zumindest teilweise in Form eines Feststoffs und/oder in Form einer Schmelze beigemengt wird.

10. Verfahren gemäß einem der Ansprüche 1 bis 5, umfassend die Schritte:
- Bereitstellen mindestens einer aus den Verbindungen mit den Formeln **II**, **III** und **IV** bestehenden Gruppe ausgewählten Verbindung, die zumindest teilweise als Schmelze vorliegt, bevorzugt Bereitstellen von Maleinsäureanhydrid, das zumindest teilweise als Schmelze vorliegt,
- Einleiten von Schwefeldioxid und Wasser in die Schmelze,
wobei beim Einleiten das Schwefeldioxid bevorzugt als Gas oder als Flüssigkeit und das Wasser bevorzugt als Dampf vorliegen und das Einleiten unter Druck erfolgt und wobei das Schwefeldioxid und das Wasser mehr bevorzugt vor dem Einleiten miteinander gemischt werden.

11. Verfahren gemäß einem der vorgenannten Ansprüche, wobei der Gehalt an Alkalimetallionen in der hergestellten Verbindung der Formel **I** höchstens 500 ppm, bevorzugt höchstens 100 ppm, mehr bevorzugt höchstens 10 ppm ist,
wobei vorzugsweise der Gehalt an Alkalimetallionen und Erdalkalimetallionen in der hergestellten Verbindung der Formel **I** insgesamt höchstens 500 ppm, bevorzugt höchstens 100 ppm, mehr bevorzugt höchstens 10 ppm ist,
wobei insbesondere der Gehalt an Kationen in der hergestellten Verbindung der Formel **I** höchstens 500 ppm, bevorzugt höchstens 100 ppm, mehr bevorzugt höchstens 10 ppm ist.

12. Verwendung einer gemäß einem der vorgenannten Ansprüche hergestellten Verbindung der Formel **I** 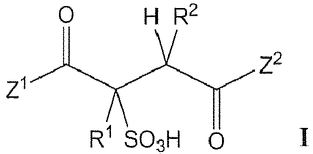 in einem aus den folgenden Verfahren (A) bis (E) ausgewählten Verfahren:
(A) Verfahren zum Herstellen eines Absorbers durch eine Vernetzung, insbesondere eine thermische Vernetzung eines Polymermaterials, wobei die Verbindung der Formel **I** als Quervernetzer verwendet wird;
(B) Verfahren zum elektrochemischen Abscheiden von Metallen und Metalllegierungen und/oder galvanotechnisches Verfahren, wobei die Verbindung der Formel **I** als Hilfsstoff verwendet wird, wobei die Verbindung der Formel **I** bevorzugt in einer Elektrolytlösung gelöst vorliegt;
(C) Verfahren zum Herstellen einer quervernetzten Membran oder einer quervernetzten Oberflächenbeschichtung durch Quervernetzung eines Polymermaterials mit einem Quervernetzer, wobei die Verbindung der Formel **I** als Quervernetzer verwendet wird, wobei es sich bei dem Polymermaterial bevorzugt um Polyvinylaklohol und/oder Cellulose I und/oder Cellulose II handelt;
(D) Verfahren zum Herstellen quervernetzter Formkörper durch Quervernetzung eines Polymermaterials mit einem Quervernetzer, wobei die Verbindung der Formel **I** als Quervernetzer verwendet wird,
wobei es sich bei dem Polymermaterial bevorzugt um ein Polysaccharid, besonders bevorzugt um Cellulose I und/oder Cellulose II, insbesondere um Fasern und/oder Kügelchen umfassend Cellulose I und/oder Cellulose II, bevorzugt Fasern und/oder Kügelchen aus Cellulose I und/oder Cellulose II handelt;
(E) Verfahren zum Herstellen eines Trägermaterials für Enzyme und/oder Mikroorganismen durch Quervernetzung eines Polymermaterials mit einem Quervernetzer, wobei die Verbindung der Formel **I** als Quervernetzer verwendet wird.

13. Verfahren zum Herstellen einer aus der aus den Verbindungen mit den Formeln **V, VI** und **VII** bestehenden Gruppe ausgewählten Verbindung, 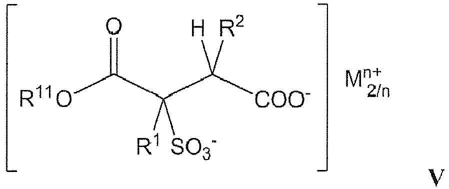 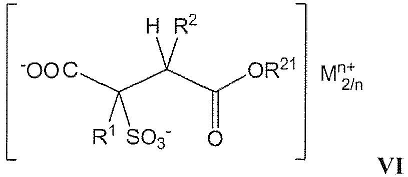 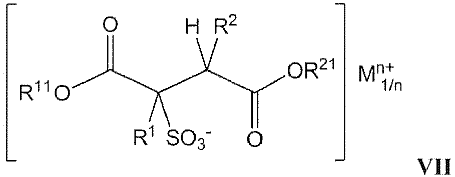 wobei -R¹, -R², -R¹¹ und -R²¹ wie in Anspruch 1 definiert sind und wobei Mⁿ⁺ ein n-wertiges Kation, insbesondere ein n-wertiges positives Metallion ist,
umfassend die folgenden Schritte:
- Durchführen eines Verfahrens gemäß einem der Ansprüche 1 bis 11 zum Herstellen der Verbindung der Formel **I;**
- Umsetzen der Verbindung der Formel **I** mit einer geeigneten Menge einer Base, die Mⁿ⁺ umfasst, insbesondere mit M(OH)ₙ, und/oder mit geeigneter geeigneten Menge von metallischem M;
- Erhalten der aus der aus den Verbindungen mit den Formeln **V, VI** und **VII** bestehenden Gruppe ausgewählten Verbindung.

14. Verwendung einer durch das Verfahren gemäß Anspruch 13 hergestellten, aus der aus den Verbindungen mit den Formeln **V, VI** und **VII** bestehenden Gruppe ausgewählten Verbindung als grenzflächenaktive Substanz.

## Geänderte Patentansprüche

### Geänderte Patentansprüche gemäss Regel 137(2) EPÜ.

1. Verfahren zum Herstellen einer Verbindung der Formel **I** 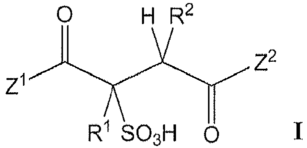 durch Umsetzen mindestens einer aus der aus den Verbindungen mit den Formeln **II, III** und **IV** bestehenden Gruppe ausgewählten Verbindung mit Schwefeldioxid in Gegenwart von Wasser, 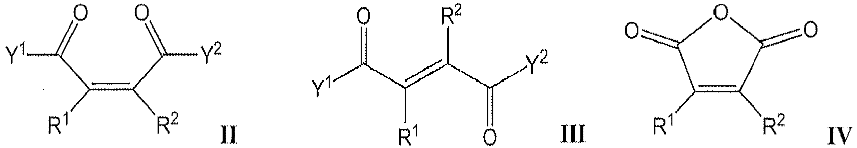
wobei -R¹ und R² unabhängig voneinander aus der aus -H und Alkyl bestehenden Gruppe ausgewählt werden,
wobei -Z¹ aus der aus -OH, -OR¹¹, -NH₂, -NHR¹², -NHR¹²R¹³ und -Cl bestehenden Gruppe ausgewählt wird,
wobei -Z² aus der aus -OH, -OR²¹, -NH₂, -NHR²², -NHR²²R²³ und -Cl bestehenden Gruppe ausgewählt wird,
wobei -Y¹ aus der aus -OH, -OR¹¹, -NH₂, -NHR¹², -NHR¹²R¹³ und -Cl bestehenden Gruppe ausgewählt wird,
wobei -Y² aus der aus -OH, -OR²¹, -NH₂, -NHR²², -NHR²²R²³ und -Cl bestehenden Gruppe ausgewählt wird,
wobei -R¹¹, -R¹², -R¹³, -R²¹, -R²², -R²³ unabhängig voneinander aus Alkylresten ausgewählt werden.

2. Verfahren gemäß Anspruch 1,
wobei R¹ und R² unabhängig voneinander aus der aus -H, Methyl, Ethyl, n-Propyl, iso-Propyl, *n*-Butyl, *iso*-Butyl, *sec*-Butyl,*tert*-Butyl und 2-Ethylhexyl bestehenden Gruppe bestehenden Gruppe ausgewählt werden, wobei bevorzugt -R¹ = -R² = -H, und/oder wobei -R¹¹, -R¹², -R¹³, -R²¹, -R²², -R²³ unabhängig voneinander aus der aus Methyl, Ethyl, *n*-Propyl, *iso*-Propyl, *n*-Butyl, *iso*-Butyl, *sec*-Butyl, *tert*-Butyl und 2-Ethylhexyl bestehenden Gruppe ausgewählt werden.

3. Verfahren gemäß einem der vorstehenden Ansprüche,
wobei -Y¹ aus der aus -OH und -OR¹¹ bestehenden Gruppe ausgewählt wird und bevorzugt -OH ist und/oder
wobei -Y² aus der aus -OH und -OR²¹ bestehenden Gruppe ausgewählt wird und bevorzugt -OH ist,
wobei -R¹¹ und -R¹² jeweils wie in Anspruch 1 definiert sind.

4. Verfahren gemäß einem der vorstehenden Ansprüche,
wobei die Verbindung mit der Formel **I** nach ihrer Herstellung in Form einer wässrigen Lösung vorliegt.

5. Verfahren gemäß einem der vorgenannten Ansprüche,
wobei die Temperatur mindestens 0 °C, bevorzugt mindestens 10 °C, noch mehr bevorzugt mindestens 20 °C beträgt und/oder
wobei bevorzugt höchstens 250 °C, mehr bevorzugt höchstens 220 °C, noch mehr bevorzugt höchstens 190 °C beträgt.

6. Verfahren gemäß einem der vorstehenden Ansprüche, umfassend die Schritte:
- Bereitstellen von Wasser;
- optional Lösen von Schwefeldioxid im Wasser;
- Beimengen mindestens einer aus der aus den Verbindungen mit den Formeln **II, III** und **IV** bestehenden Gruppe ausgewählten Verbindung;
- wobei -R¹, -R², -Y¹ und -Y² wie in Anspruch 1 definiert sind;
- Einleiten von Schwefeldioxid.

7. Verfahren gemäß Anspruch 6,
wobei das Schwefeldioxid in Form von Schwefeldioxidgas und/oder flüssigem Schwefeldioxid eingeleitet wird,
wobei das Einleiten bevorzugt unter Überdruck erfolgt,
wobei das Verhältnis der Stoffmenge des eingesetzten Schwefeldioxids und der Stoffmenge der eingesetzten aus den Verbindungen mit den Formeln **II, III** und **IV** bestehenden Gruppe ausgewählten Verbindung bevorzugt höchstens 1000, mehr bevorzugt höchstens 500, noch mehr bevorzugt höchstens 100 und/oder bevorzugt mindestens 1, mehr bevorzugt mindestens 5, noch mehr bevorzugt mindestens 10 ist.

8. Verfahren gemäß einem der Ansprüche 6 oder 7,
wobei die mindestens eine aus der aus den Verbindungen mit den Formeln **II, III** und **IV** bestehenden Gruppe ausgewählte Verbindung zumindest teilweise in Form einer wässrigen Lösung beigemengt wird.

9. Verfahren gemäß einem der Ansprüche 6 bis 8,
wobei die mindestens eine aus der aus den Verbindungen mit den Formeln **II, III** und **IV** bestehenden Gruppe ausgewählte Verbindung zumindest teilweise in Form eines Feststoffs und/oder in Form einer Schmelze beigemengt wird.

10. Verfahren gemäß einem der Ansprüche 1 bis 5, umfassend die Schritte:
- Bereitstellen mindestens einer aus den Verbindungen mit den Formeln **II, III** und **IV** bestehenden Gruppe ausgewählten Verbindung, die zumindest teilweise als Schmelze vorliegt, bevorzugt Bereitstellen von Maleinsäureanhydrid, das zumindest teilweise als Schmelze vorliegt,
- Einleiten von Schwefeldioxid und Wasser in die Schmelze,
wobei beim Einleiten das Schwefeldioxid bevorzugt als Gas oder als Flüssigkeit und das Wasser bevorzugt als Dampf vorliegen und das Einleiten unter Druck erfolgt und wobei das Schwefeldioxid und das Wasser mehr bevorzugt vor dem Einleiten miteinander gemischt werden.

11. Verfahren gemäß einem der vorgenannten Ansprüche, wobei der Gehalt an Alkalimetallionen in der hergestellten Verbindung der Formel **I** höchstens 500 ppm, bevorzugt höchstens 100 ppm, mehr bevorzugt höchstens 10 ppm ist,
wobei vorzugsweise der Gehalt an Alkalimetallionen und Erdalkalimetallionen in der hergestellten Verbindung der Formel **I** insgesamt höchstens 500 ppm, bevorzugt höchstens 100 ppm, mehr bevorzugt höchstens 10 ppm ist,
wobei insbesondere der Gehalt an Kationen in der hergestellten Verbindung der Formel **I** höchstens 500 ppm, bevorzugt höchstens 100 ppm, mehr bevorzugt höchstens 10 ppm ist.

12. Verwendung einer Verbindung der Formel **I** 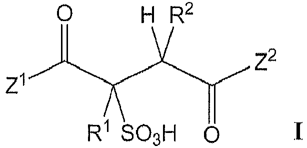 in einem aus den folgenden Verfahren (A) bis (E) ausgewählten Verfahren,
wobei die Verbindung der Formel **I** nach einem Verfahren gemäß einem der vorgenannten Ansprüche hergestellt worden ist:
(A) Verfahren zum Herstellen eines Absorbers durch eine Vernetzung, insbesondere eine thermische Vernetzung eines Polymermaterials, wobei die Verbindung der Formel **I** als Quervernetzer verwendet wird;
(B) Verfahren zum elektrochemischen Abscheiden von Metallen und Metalllegierungen und/oder galvanotechnisches Verfahren, wobei die Verbindung der Formel **I** als Hilfsstoff verwendet wird, wobei die Verbindung der Formel **I** bevorzugt in einer Elektrolytlösung gelöst vorliegt;
(C) Verfahren zum Herstellen einer quervernetzten Membran oder einer quervernetzten Oberflächenbeschichtung durch Quervernetzung eines Polymermaterials mit einem Quervernetzer, wobei die Verbindung der Formel **I** als Quervernetzer verwendet wird, wobei es sich bei dem Polymermaterial bevorzugt um Polyvinylaklohol und/oder Cellulose I und/oder Cellulose II handelt;
(D) Verfahren zum Herstellen quervernetzter Formkörper durch Quervernetzung eines Polymermaterials mit einem Quervernetzer, wobei die Verbindung der Formel **I** als Quervernetzer verwendet wird,
wobei es sich bei dem Polymermaterial bevorzugt um ein Polysaccharid, besonders bevorzugt um Cellulose I und/oder Cellulose II, insbesondere um Fasern und/oder Kügelchen umfassend Cellulose I und/oder Cellulose II, bevorzugt Fasern und/oder Kügelchen aus Cellulose I und/oder Cellulose II handelt;
(E) Verfahren zum Herstellen eines Trägermaterials für Enzyme und/oder Mikroorganismen durch Quervernetzung eines Polymermaterials mit einem Quervernetzer, wobei die Verbindung der Formel **I** als Quervernetzer verwendet wird.

13. Verfahren zum Herstellen einer aus der aus den Verbindungen mit den Formeln **V, VI** und **VII** bestehenden Gruppe ausgewählten Verbindung, 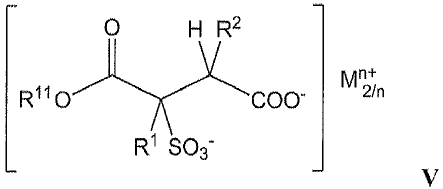 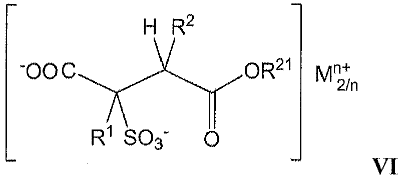 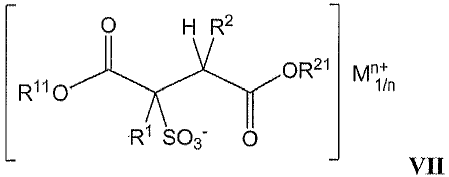 wobei -R¹, -R², -R¹¹ und -R²¹ wie in Anspruch 1 definiert sind und wobei Mⁿ⁺ ein n-wertiges Kation, insbesondere ein n-wertiges positives Metallion ist,
umfassend die folgenden Schritte:
- Durchführen eines Verfahrens gemäß einem der Ansprüche 1 bis 11 zum Herstellen der Verbindung der Formel **I;**
- Umsetzen der Verbindung der Formel **I** mit einer geeigneten Menge einer Base, die Mⁿ⁺ umfasst, insbesondere mit M(OH)ₙ, und/oder mit geeigneter geeigneten Menge von metallischem M;
- Erhalten der aus der aus den Verbindungen mit den Formeln **V, VI** und **VII** bestehenden Gruppe ausgewählten Verbindung.

14. Verwendung einer gemäß Anspruch 13 hergestellten, aus der aus den Verbindungen mit den Formeln **V, VI** und **VII** bestehenden Gruppe ausgewählten Verbindung als grenzflächenaktive Substanz, wobei die aus der aus den Verbindungen mit den Formeln **V, VI** und **VII** bestehenden Gruppe ausgewählte Verbindung durch ein Verfahren gemäß Anspruch 13 hergestellt worden ist.
